# EUROPEAN PATENT SPECIFICATION

(11) **EP 1 173 172 B1**
(45) Date of publication and mention of the grant of the patent: **04.01.2006**
(21) Application number: 00928200.5
(22) Date of filing: 18.04.2000
(51) Int. Cl.: A61K 31/40, A61K 31/4418, A61K 31/4422, A61P 3/06, A61P 9/10, A61P 9/12

(54) **SYNERGISTIC EFFECTS OF AMLODIPINE AND THE HYDROXYLATED METABOLITE OF ATORVASTATIN**
SYNERGISTISCHE WIRKUNGEN VON AMLODIPIN UND DEM HYDROXYLIERTEN METABOLIT VON ATORVASTATIN
EFFETS SYNERGIQUES DE L'AMLODIPINE ET DU METABOLITE HYDROXYLE DE L'ATORVASTATINE

(30) Priority: 23.04.1999 US 130665 P; 23.07.1999 US 145305 P; 27.08.1999 US 151121 P; 19.11.1999 US 166592 P
(43) Date of publication of application: 23.01.2002
(73) Proprietor: Mason, R. Preston, Manchester, MA 01944 (US)
(72) Inventor: Mason, R. Preston, Manchester, MA 01944 (US)
(74) Representative: Duffy, Assumpta Dympna
(86) International application number: PCT/US2000/010465
(87) International publication number: WO 2000/064443

(56) References cited:
- WO-A1-99/11259
- US-A- 5 385 929
- AVIRAM M., ROSENBLAT M., BISGAIER C. L., NEWTON R.S.: "Atorvastatin and gemfibrozil metabolites, but not the parent drugs, are potent antioxidants against lipoprotein oxidation" ATHEROSCLEROSIS, vol. 138, no. 2, 1998, pages 271-280, XP002198850
- JUKEMA J W ET AL: "EVIDENCE FOR A SYNERGISTIC EFFECT OF CALCIUM CHANNEL BLOCKERS WITH LIPID-LOWERING THERAPY IN RETARDING PROGRESSION OF CORONARY ATHEROSCLEROSIS IN SYMPTOMATIC PATIENTS WITH NORMAL TO MODERATELY RAISED CHOLESTEROL LEVELS" ARTERIOSCLEROSIS, THROMBOSIS, AND VASCULAR BIOLOGY, XX, XX, vol. 16, no. 3, March 1996 (1996-03), pages 425-430, XP000997305 ISSN: 1079-5642
- OREKHOV A N ET AL: "antiatherosclerotic and antiatherogenic effects of a calcium antagonist plus statin combination: Amlodipine and Lovastatin" CARDIOVASCULAR DRUGS AND THERAPY, KLUWER ACADEMIC PUBLISHERS, BOSTON, US, vol. 11, no. 2, 1 June 1997 (1997-06-01), page 350 XP002084181 ISSN: 0920-3206

## Description

### Cross Reference to Related Applications

This application claims priority from the following four provisional patent applications: U.S. Application No. 60/130,665, filed on April 23, 1999; U.S. Application No. 60/145,305, filed on July 23, 1999; U.S. Application No. 60/151,121, filed on August 27, 1999; and U.S. Application No. 60/166,592, filed on November 19, 1999, and is a continuation-in-part application of our U.S. application Ser. No. 09/556,930 filed April 21, 2000.

### Field of Invention

The present invention relates to pharmaceutical compositions and combinations to treat arterial and related heart disease and related ailments.

### Background of the Invention

Coronary artery disease (CAD) is the leading cause of mortality in the developed world, and is associated with substantial morbidity as well. Typically, the patient with CAD has several concomitant conditions, including hypertension, diabetes, and dyslipidemia, increasing overall risk for poor outcomes and complicating treatment.

Among antihypertensive therapies, the lipophilic dihydropyridine-type calcium channel blocker (CCB) amlodipine besylate (AML) is a very well-tolerated agent with an established record of safety and effectiveness for the treatment of hypertension and angina. A potential therapeutic role for AML in the treatment of patients with CAD was recently shown in the Prospective Randomized Evaluation of the Vascular Effects of Norvasc® (AML) Trial (PREVENT). This three-year trial evaluated the effects of AML compared to placebo on the development and progression of atherosclerotic lesions in coronary and carotid arteries among patients with documented CAD (Byington RP, Miller ME, Herrington D, et al. Rationale, design, and baseline characteristics of the Prospective Randomized Evaluation of the Vascular Effects of Norvasc Trial (PREVENT). *Am. J. Cardiol.* 1997; 80:1087-1090). The results of PREVENT showed impressive clinical benefits with AML therapy, including an overall 30% reduction in major documented events or procedures (Byington RP, Chen J, Furberg CD, Pitt B. Effect of amlodipine on cardiovascular events and procedures. *J.* Am. *Coll. Cardiol.* 1999; 33:314A and Pitt B, Byington RP, Hunninghake DB, Mancini J, Miller ME, Riley W. Effect of amlodipine on the progression of atherosclerosis and occurrence of clinical events. *Circulation* 2000; 102:1503-1510). AML therapy was also associated with a significant slowing in the progression of carotid atherosclerosis, as measured by B-mode ultrasonographic assessments (Byingyton R, Riley W, Booth D, et al. Effect of amlodipine on progression of carotid atherosclerosis in patients with documented heart disease. *Am. J. Hypertens.* 1999; 12:42A-43A and Pitt B, Byington RP, Hunninghake DB, Mancini J, Miller ME, Riley W. Effect of amlodipine on the progression of atherosclerosis and occurrence of clinical events. Circulation 2000; 102:1503-1510). The clinical benefit seen with AML in CAD has not been previously reported with other CCBs, including dihydropyridine-type agents that have been used to examine this question (Waters D, Lesperance J, Francetich M, et al. A controlled clinical trial to assess the effect of a calcium channel blocker on the progression of coronary atherosclerosis. *Circulation* 1990; 82:1940-1953; Lichtlen PR, Hugenholtz PG, Rafflenbeul W, et al. Retardation of coronary artery disease in humans by the calcium-channel blocker nifedipine: Results of the INTACT study (International Nifedipine Trial on Antiatherosclerotic Therapy). *Cardiovasc. Drugs Ther.* 1990; 4:S1047-S1068; Borhani NO, Mercuri M. Borhani PA, et al. Final outcome results of the Multicenter Isradipine Diuretic Atherosclerosis Study (MIDAS). A randomized controlled trial. JAMA 1996; 276:785-791). This observation has led to interest in potential antiatherogenic properties of AML, including antioxidant effects that are independent of calcium channel modulation (Mason RP, Leeds PR, Jacob RE, et al. Inhibition of excessive neuronal apoptosis by the calcium antagonist amlodipine and antioxidants in cerebellar granule cells. *J. Neurochem.* 1999; *72*:1448-*1456;* Tulenko TN, Laury-Kleintop L, Walter MF, Mason RP. Cholesterol, calcium and atherosclerosis: Is there a role for calcium channel blockers in atheroprotection? *Int. J. Cardiol*. 1997; 62 (2 Suppl):55S- 66S; Kramsch DM, Sharma RC. Limits of lipid-lowering therapy: The benefits of amlodipine as an anti-atherosclerotic agent. *J. Hum. Hypertens. 1995;* 9 (Suppl 1:S3-S9); and Mason RP, Walter MF, Trumbore MW, Olmstead EG, Mason PE. Membrane antioxidant effects of the charged dihydropyridine calcium antagonist amlodipine. *J. Mol. Cell. Cardiol* 1999; 31:275-281.

Hypolipidemic therapy has also been demonstrated to be very useful in reducing morbidity and mortality associated with CAD. The ortho- and para- hydroxylated metabolites of atorvastatin ATM have been shown to exhibit antioxidant effects in lipoprotein preparations (Aviram M, Rosenblat M, Bisgaier CL, Newton RS. Atorvastatin and gemfibrozil metabolites but not the parent drugs, are potent antioxidants against lipoprotein oxidation. *Atherosclerosis* 1998: 138:271-280). The ortho-, meta-, and para-hydroxylated metabolites of atorvastatin (ATM) and their methods of preparation are shown in U.S. Patent 5,385,929.

However, no pharmaceutical composition currently exists that treats both hypertension and hyperlipidemia. Such a pharmaceutical composition would have several benefits. For example, the multiple risk factors for arterial and related heart disease that are often present in an individual patient could be targeted simultaneously. Additionally, the ease of taking one combined dosage could significantly enhance patient compliance with therapeutic regimens.

Therefore, it is an object of this invention to provide a combination therapy that will treat the multiple pathological processes involved in arterial and related heart disease.

These include, but are not limited to, hypertension and hyperlipidemia. It is also an object of this invention to develop useful and convenient dosage levels and forms of such a combination therapeutic. Preferably, this pharmaceutical composition would have synergistic effects on these hallmarks of arterial and related heart disease, such that the individual effects of the components of this composition would be enhanced by their combination.

Thus, this invention encompasses a therapeutic goal for the treatment of CAD that entails the development of drugs that can simultaneously target multiple underlying disease processes that contribute to atherosclerosis, thereby altering the course of the disease. Therefore, using this invention, CAD therapy may have increased positive outcomes if the use of an antihypertensive agent and HMG-CoA reductase inhibitor was combined in a single delivery system.

### Summary of the Invention

Unexpectedly, when AML and ATM were combined, they had a synergistic effect in preventing lipid peroxidation in human low-density lipoproteins (LDL) and lipid membranes. The activity of the combination is considered synergistic as the measured effect significantly exceeded any additive effects of the two drugs. Therefore, these agents have heretofore unrecognized synergistic antioxidant effects, a property that would enable these agents to increase the resistance of LDL and vascular cell membranes to oxidative modification during atherogenesis. Indeed, oxidative modification of lipids is a well-established cause of injury to the endothelium and underlying smooth muscle (Ross R. Atherosclerosis . An inflammatory disease. *N. Engl. J. Med.* 1999; 340:115-126; Diaz MN, Frei B, Vita JA, Keaney JF. Antioxidants and atherosclerotic heart disease. *N. Engl. J. Med*. 1997; 337:408-416). Lipophilic agents that protect against lipid peroxidation have been shown to reduce lesion development in various models of atherosclerosis as well as clinical studies (Diaz MN, Frei B, Vita JA, Keaney JF. Antioxidants and atherosclerotic heart disease. *N Engl. J. Med.* 1997; 337:408-416). Moreover, the benefit associated with hypolipidemic therapy is attributed to both its effect on plasma very low-density lipoproteins (VLDL), LDL, and high-density lipoproteins (HDL) levels and, as a consequence, to a reduction in the potential formation of atherogenic oxidized lipoproteins.

Scientific analyses that support the combined use of AML (Norvasc®) and ATM in a single delivery system for the treatment of cardiovascular disease are described in this invention. Specifically, the synergistic antioxidant activities of the calcium channel blocker, AML, and the active hydroxylated metabolite of the HMG-CoA reductase inhibitor atorvastatin, ATM were evaluated in human LDL and lipid membranes enriched with polyunsaturated fatty acids (PUFA), the key target for oxy-radical damage in atherosclerosis. The synergistic effects of these agents were demonstrated in membranes prepared in the presence of cholesterol. The combination of AML with ATM effected a dramatic and sustained reduction in lipid oxy-radical damage at concentrations as low as 10.0 nM. The dose-dependent antioxidant activity associated with the combination of these drugs at therapeutic levels was highly synergistic and could not be effectively reproduced by the endogenous agent, vitamin E. Antioxidant activity was not observed, however, when AML was combined with other HMG-CoA reductase inhibitors, including lovastatin and mevastatin. As determined by x-ray diffraction and chemical analyses, the distinct activity described for this drug combination can be attributed to strong physico-chemical interactions with the membrane bilayer that are independent of the well-characterized effects of these drugs on calcium transport and cholesterol metabolism. This synergistic antioxidant benefit constitutes a new pharmacologic mechanism of action for these compounds and a compelling rationale for the combined use of the active ingredients in Norvasc® and ATM in the treatment of cardiovascular disease by reducing the levels of LDL in plasma and improving protection of LDL and cellular membranes against oxidation. This new property complements the established effects of these drugs on hypertension and dyslipidemia.

Other objects, features, and advantages of the present invention will be apparent from the following Detailed Description of the Preferred Embodiments taken in conjunction with the accompanying drawings in which:

### Brief Description of the Drawings

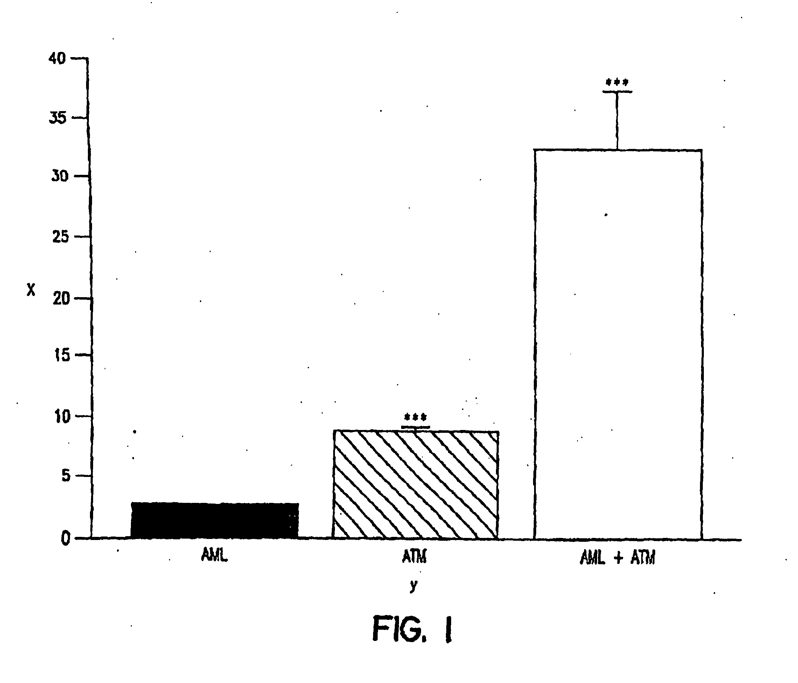

Figure 1 shows the synergistic effects of AML and ATM on lipid peroxidation at a low, therapeutic concentration of drug (100.0 nM) in membranes containing physiologic levels of cholesterol. x is percent inhibition of lipid peroxidation and y is treatment with amlodipine (AML), atorvastatin metabolite (ATM), and the combination of both (AML +ATM) at a level of 100 nM. Values are mean ± standard deviation for n 6. ··· indicates p<0.001 versus control and other treatments.

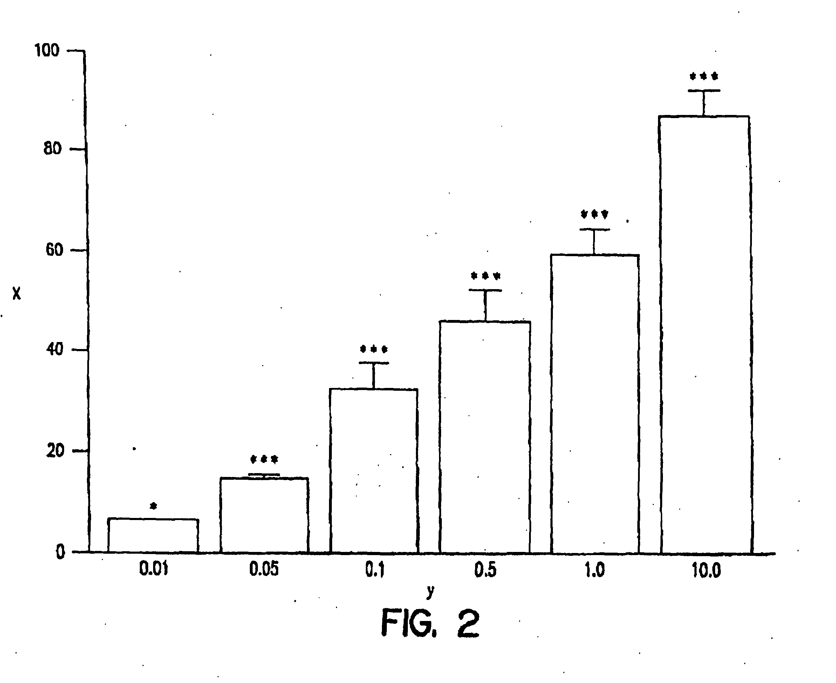

Figure 2 shows the dose-dependent antioxidant effect of the combination of AML and ATM over a broad range of concentrations (0.01 through 10.0 µM). x is percent inhibition of lipid peroxidation and y is treatment with the combination of AML and ATM at the micromolar concentrations indicated. Values are mean ± standard deviation for n =6.*** indicates p<0.001 versus control and other treatments.

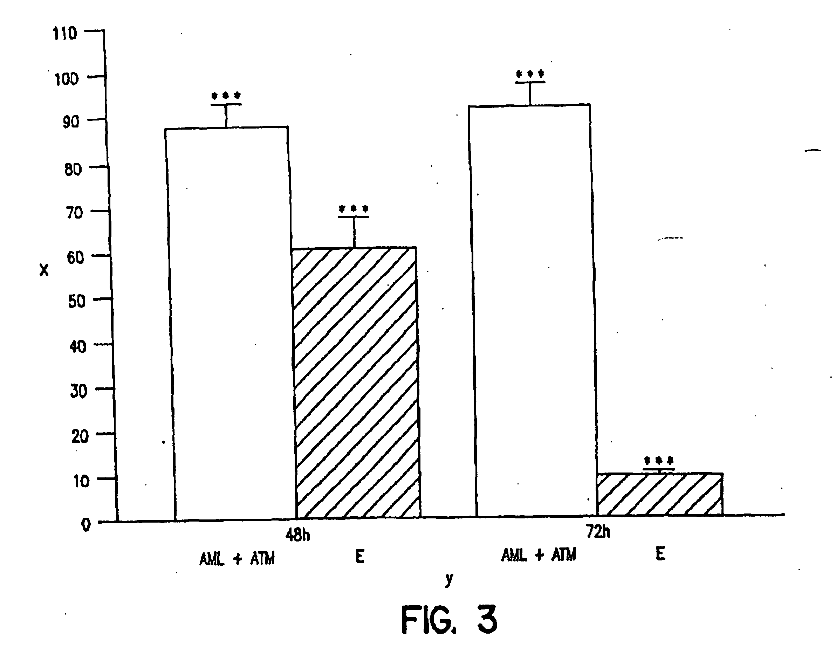

Figure 3 shows the superior antioxidant activity of the AML/ATM combination over vitamin E as a function of time at an identical concentration (10.0 µM). x is percent inhibition of lipid peroxidation and y is treatment with the combination of amlodipine and atorvastatin metabolite (AML + ATM), darkened panel, or vitamin E (E), cross-hatched panel, both at 10 µM. Values are mean ± standard deviation for n=6 to 12. ***. indicates p<0.001 versus control and other treatments.

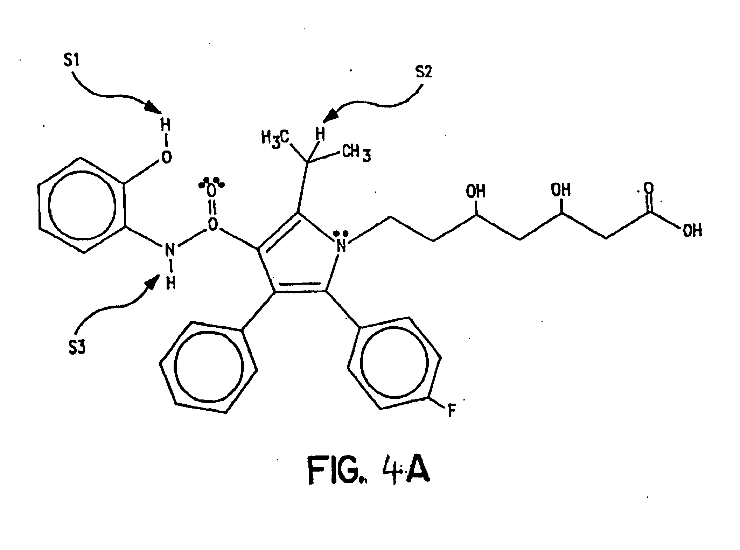

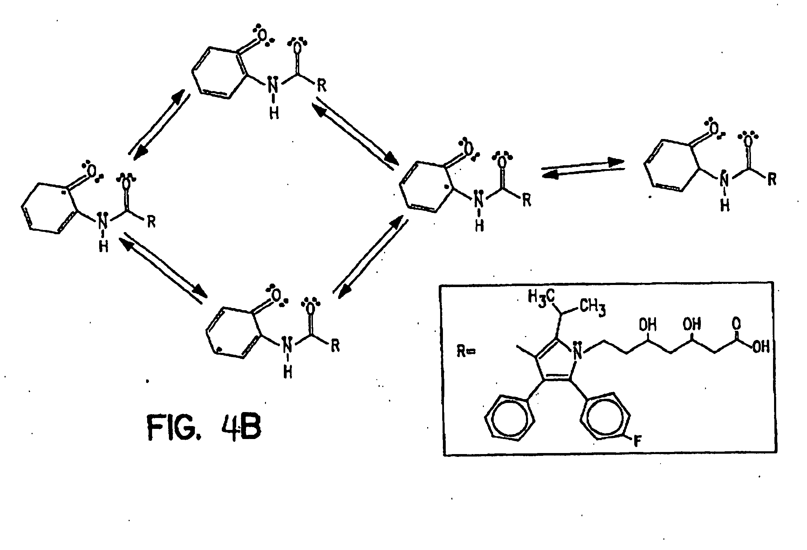

Figures 4A-4D show the sites of proton abstraction (S1, S2, and S3) for ATM that contribute to antioxidant activity in Figure 4A along with resonance stabilization calculations (Figures 4B through 4D).

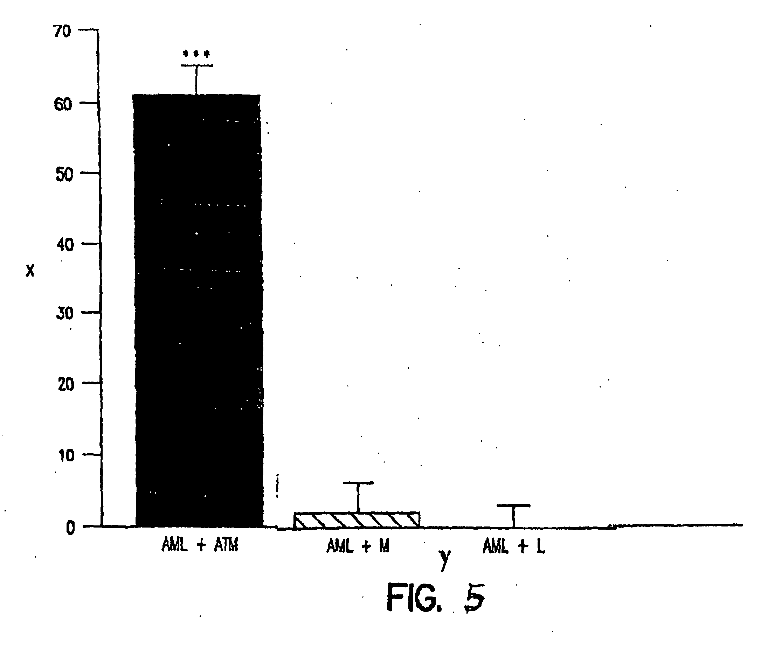

Figure 5 shows the comparative effects of AML antioxidant activity when combined with different HMG-CoA reductase inhibitors at the same concentration. x is percent inhibition of lipid peroxidation and y is treatment with the combination of amlodipine and atorvastatin metabolite (AML + ATM), darkened panel; amlodipine and mevastatin (AML+M), cross-hatched panel; or amlodipine and lovastatin (AML +L), no lipid peroxidation inhibition; all at 1.0 µM. Values are mean ± standard deviation for n=6 to 12.***. indicates p<0.001 versus control and other treatments.

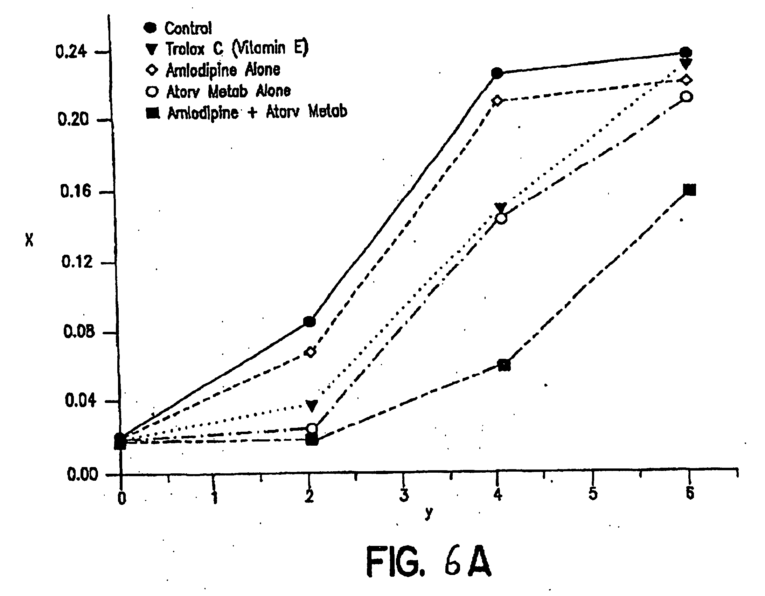

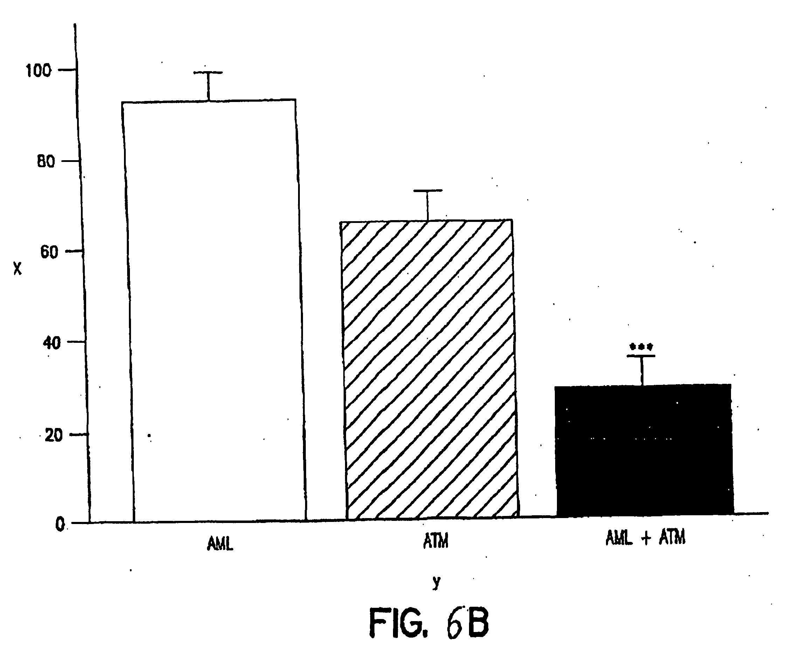

Figures 6A-6B show the synergistic antioxidant effects of AML and ATM in human LDL samples, as compared to Trolox C (soluble vitamin E). In (Figure 6A), x is thiobarbituric reactive substances (TBARS) formation measured at an absorbance of 532 nm and y is time in hours. The control is indicated by filled circles with solid lines, Trolox C (soluble vitamin E) is indicated by filled, inverted triangles with small dashed lines, AML alone is indicated by open diamonds with long dashed lines, ATM alone is indicated by open circles with alternating long and short dashed lines, and AML and ATM is indicated by filled squares with dotted lines. In (Figure 6B), x is thiobarbituric reactive substances (TBARS) formation measured at an absorbance of 532 nm and y is treatment with the combination of amlodipine (AML), darkened panel; atorvastatin metabolite (ATM), cross-hatched panel; or amlodipine and atorvastatin metabolite (AML+ ATM), darkened panel, at a concentration of 3.0 µM. Values are mean + standard deviation.*** indicates p<0.001 versus control and other treatments.

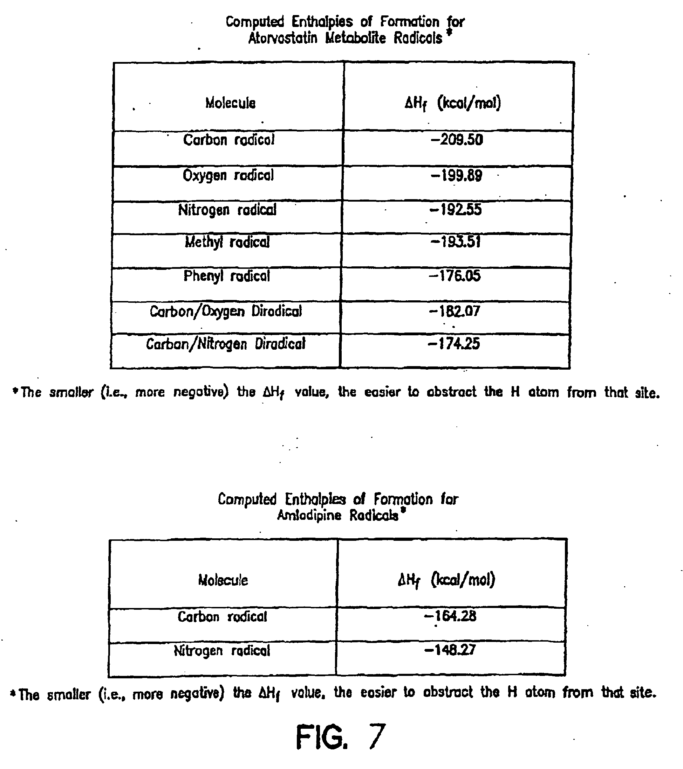

Figure 7 shows the computed enthalpies for ATM, AML and their respective radical species.

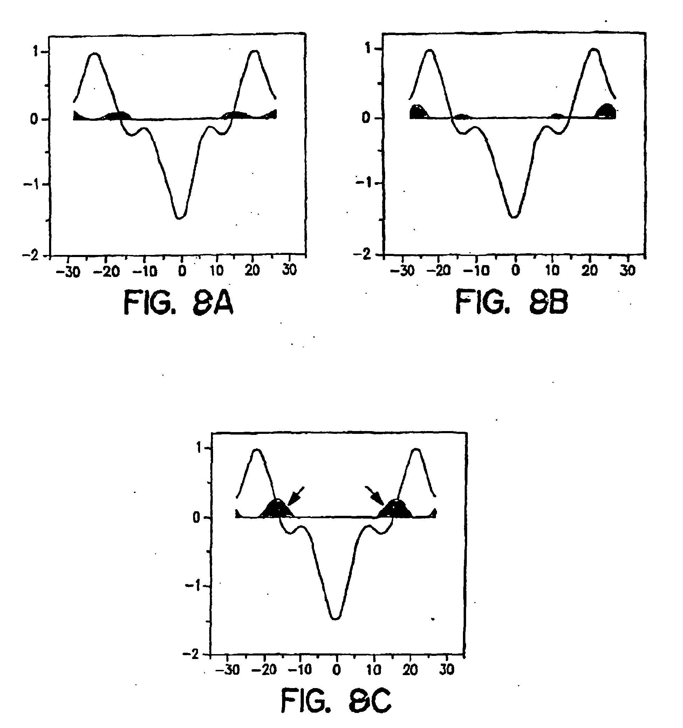

Figures 8A-8C show x-ray diffraction determination of the separate versus combined lipid membrane interactions of AML and ATM. x is relative electron density and y is angstroms from the middle of the lipid bilayer. In all three panels the control is the solid line. In Figure 8A, AML is the dotted line; in Figure 8B, ATM is the dotted line; in Figure 8C, the combination of AML and ATM is the dotted line. The darkened areas in each panel are the difference in electron densities between the control and treatment electron density profiles.

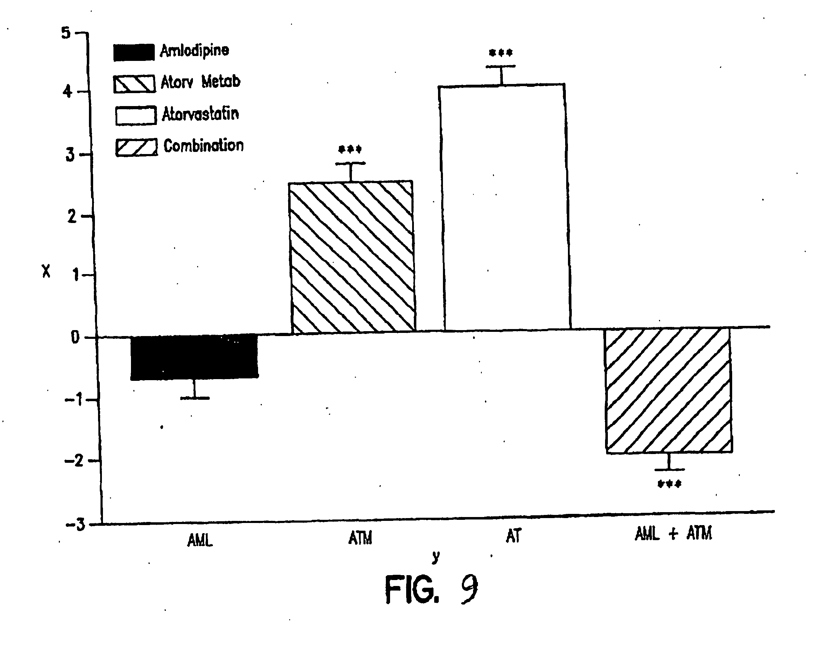

Figure 9 shows the separate versus combined effects of AML and ATM on membrane bilayer dimensions as determined by x-ray diffraction analysis. x is the change in membrane width in angstroms and y is the treatment with AML, darkened panel; ATM, cross-hatched panel; and the combination of AML and ATM, hatched panel. Values are mean ± standard deviation. *** indicates p<0.001 versus control and other treatments.

### Detailed Description of Embodiments

*Synergistic Antioxidant Effects of Amlodipine with Atorvastatin Metabolite in Lipid Membranes:* The separate and combined dose-dependent antioxidant activities of AML, and ATM were tested in membrane vesicles reconstituted from phospholipids enriched with cholesterol and the PUFA, dilinoleoyl phosphatidylcholine, at a 0.5:1 mole ratio. Membrane vesicles were used in these experiments for the following reasons: 1) this system is well-defined and highly reproducible; 2) linoleic acid represents the primary target for oxidative damage and is common in vascular cell membranes and lipoprotein particles; 3) this membrane system does not contain calcium channels or the HMG CoA reductase enzyme and; 4) lipid peroxidation in this system can be initiated spontaneously at 37°C in the absence of exogenous chemical initiators, such as high levels of iron and ascorbate. In these experiments, oxidation occurred in a gradual, time-dependent manner that was measured spectrophotometrically over a 72 h period.

In Figure 1, the synergistic antioxidant activity of AML and ATM was demonstrated in membrane vesicles composed of cholesterol and phospholipid at levels that reproduce physiologic-like conditions (Tulenko TN, Chen M, Mason PE, Mason RP. Physical effects of cholesterol on arterial smooth muscle membranes: Evidence of immiscible cholesterol domains and alterations in bilayer width during atherogenesis. *J. Lipid. Res.* 1998; 39:947- 956). At 100.0 nM, only the ATM separately produced any significant inhibition (9% of control) of lipid peroxidation in this membrane preparation enriched with cholesterol. When the agents were combined, however, the extent of inhibition increased to 33%, an effect significantly (p<0.01) greater than that measured for the agents separately. The antioxidant activity of the combination was very apparent: the drugs inhibited lipid peroxide formation (>5 x 10² µM) at a concentration of 100.0 nM (the contro 1 level of lipid peroxide formation was 1.6 mM). This drug combination produced an effect that was highly dose-dependent over a broad range of concentrations (Figure 2). Significant inhibition (p<0.05) was observed as low as 10.0 nM with an IC₅₀ of 500.0 µM. Greater than 90% inhibition (>1 mM lipid peroxide formation) was observed at a concentration of 10.0 µM for the combination (Figure 2). The fact that inhibition was observed at submicromolar levels indicates that the benefit observed with the combination of AML and ATM is of therapeutic relevance.

The antioxidant effect of the combination persisted over time in a manner that could not be reproduced by vitamin E, even at an elevated concentration (10.0 µM) (Figure 3). This observation is consistent with the concept that vitamin E or α-tocopherol is gradually consumed during the lipid peroxidation process. By contrast, the activity of the AML/ATM combination was not affected by the length of the incubation period in which the total lipid peroxide level increased to 2.2 mM at the 72 h time point.

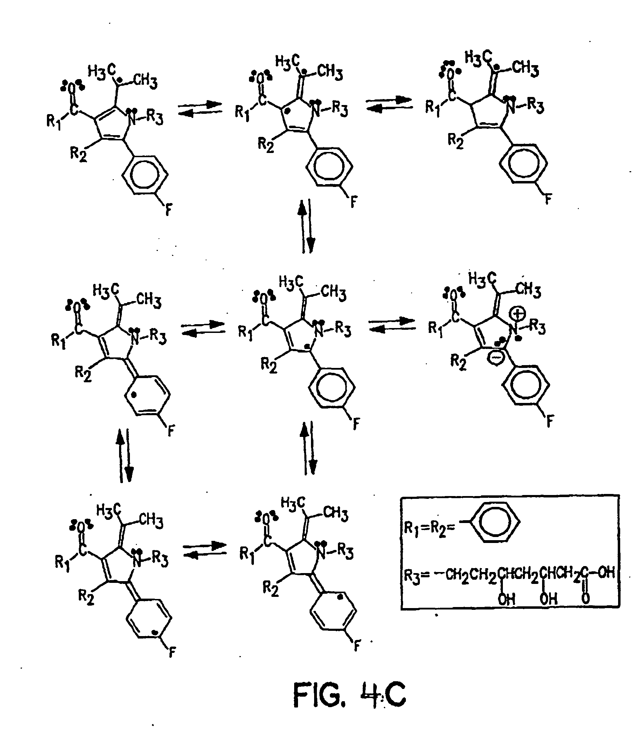

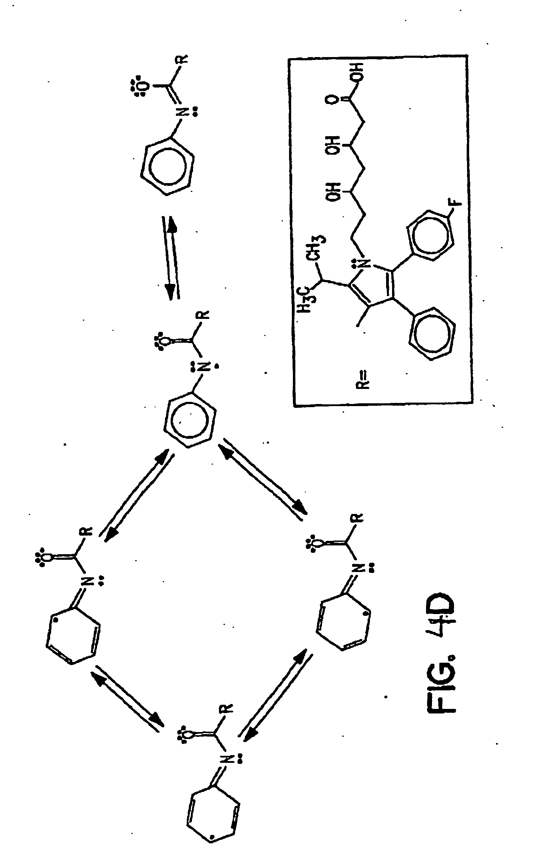

The increased polarity of ATM, mediated by its additional hydroxy group, may facilitate stronger interactions with the formally charged AML, leading to distinct interactions with phospholipid molecules, as evidenced by x-ray diffraction analysis. The additional hydroxy group associated with the ATM also provides an additional abstractable proton that can be donated to free radical molecules (Figure 4). Following the loss of the proton, the remaining unpaired free electron can be effectively stabilized in resonance structures of the metabolite, as shown in Figure 4. The distinct antioxidant activity of the combination of AML with ATM is indicated by the observation that a similar effect could not be reproduced when AML was combined with each of two other statins (mevastatin and lovastatin), as demonstrated in Figure 5.

The results of *in vivo* investigations have indicated an important role for lipophilic antioxidants in reducing cardiovascular morbidity and mortality, especially CAD. LDL particles that have greater resistance to oxidative damage exhibit reduced cytotoxicity, interfere less with endothelium-derived nitric oxide production, and do not contribute to foam cell formation (Diaz MN, Frei B, Vita JA, Keaney JF.

Antioxidants and atherosclerotic heart disease. *N.*

*Engl. J. Med.* 1997; 337:408-416). Supplementation with an antioxidant has been shown to increase LDL resistance to oxidative modification and reduce endothelial cell cytotoxicity (Belcher JD, Balla J, Balla G, et al. Vitamin E, LDL, and endothelium. Brief oral vitamin supplementation prevents oxidized LDL-mediated vascular injury in vitro. *Arterioscler. Thromb.* 1993; 13:1779-1789). Probucol, a lipophilic antioxidant, attenuated the formation of atherosclerotic plaques in cholesterol-fed primates, an effect that correlated with increased resistance of LDL to oxidative damage (Sasahara M, Raines EW, Chait A, et al. Inhibition of hypercholesterolemia-induced atherosclerosis in the nonhuman primate by probucol. I. Is the extent of atherosclerosis related to resistance of LDL to oxidation? *J. Clin. Invest.* 1994; 94:155-164). This antioxidant inhibited the formation of lesions in Watanabe hereditary hyperlipidemic (WHHL) rabbits, a well-characterized animal model of atherosclerosis, independent of cholesterol-lowering effects (Carew TE, Schwenke DC, Steinberg D. Antiatherogenic effect of probucol unrelated to its hypocholesterolemic effect: Evidence that antioxidants in vivo can selectively inhibit low density lipoprotein degradation in macrophage-rich fatty streaks and slow the progression of atherosclerosis in the Watanabe heritable hyperlipidemic rabbit. *Proc. Natl. Acad. Sci. USA* 1987; 84:7725-7729). Beyond these animal studies, a placebo-controlled clinical study demonstrated that probucol reduced restenosis by 47% in patients with CAD following coronary-artery balloon angioplasty, presumably due to its antioxidant effects (Tardif JC, Cote G, Lesperance J, et al. Probucol and multivitamins in the prevention of restonosis after coronary angioplasty. Multivitamins and Probucol Study Group. N *Engl. J. Med.* 1997; 337:365-372). In a separate study, it was demonstrated that probucol, unlike antioxidant vitamins, had a beneficial effect on vascular remodeling in patients that had underwent angioplasty, as determined by intravascular ultrasound techniques (Cote G, Tardif J-C, Lesperance J, et al. Effects of probucol on vascular remodeling after coronary angioplasty. Circulation 1999; 99:30-35).

Thus, a review of the available data provides a mechanistic rationale for the use of lipophilic antioxidants to interfere with inflammatory processes associated with CAD. By increasing the resistance of LDL and vascular cell membranes to oxidative damage, agents with antioxidant activity may effectively interfere with pathologic alterations in the vessel wall during atherogenesis. These processes include, but are not limited to, foam cell formation, endothelial dysfunction and toxicity, leukocyte and platelet adhesion, and arterial vasospasm, secondary to a loss of normal nitric oxide production. These cellular observations support epidemiologic analyses that indicate an inverse association between the serum levels of certain antioxidants and adverse outcomes associated with coronary disease (Stampfer MJ, Hennekens CH, Manson JE, Colditz GA, Rosner B, Willett WC. Vitamin E consumption and the risk of coronary disease in women. *N. Engl.* J. *Med* 1993; 328:1450-1456; Rimm EB, Stampfer MJ, Ascherio A, Giovannucci E, Colditz GA, Willett WC. Vitamin E consumption and the risk of coronary heart disease in men. *N Engl. J. Med.* 1993; 328:1450-1456; Enstrom JE, Kanim LE, Klein MA. Vitamin C intake and mortality among a sample of the United States population. Epidemiology 1992; 3:194-202; Riemersma RA, Wood DA, Macintyre CC, Elton R, Gey KF, Oliver MF. Low plasma vitamins E and C. Increased risk of angina in Scottish men. *Ann. N Y. Acad Sci.* 1989; 570:291-295; Ramirez J, Flowers NC. Leukocyte ascorbic acid and its relationship to coronary artery disease in man. Am. *J. Clin. Nutr.* 1980; 33:2079-2087; Hennekens CH, Buring JE, Manson JE, et al. Lack of effect of long-term supplementation with beta carotene on the incidence of malignant neoplasms and cardiovascular disease. *N. Engl. J. Med* 1996; 334:1145-1149; Losonczy KG, Harris TB, Havlik RJ. Vitamin E and vitamin C supplement use and risk of all-cause and coronary heart disease mortality in older persons: The Established Populations for Epidemiologic Studies of the Elderly. *Am. J. Clin. Nutr.* 1996; 64:190-196). Several of these epidemiologic studies showed benefit with vitamin E, an antioxidant with limited capacity to interfere with oxidative modification. The results of this study would predict that the combination of AML and ATM would be significantly more effective than vitamin E in reducing vascular injury associated with CAD.

*Synergistic Antioxidant Effects of Amlodipine with the Hydroxy Atorvastatin Metabolite in Human LDL:* The separate and combined antioxidant effects of AML and ATM were also evaluated in human LDL preparations. The ability of these agents to inhibit LDL peroxidation was assessed in vitro following addition of copper (10.0 µM) by measuring the levels of thiobarbituric reactive substances (TBARS), a marker of lipid peroxidation. Figure 6 shows that the rate of LDL oxidation was characterized by sigmoidal curve kinetics with an initial lag phase followed by a sharp propagation and final plateau phase (Esterbauer H, Gebicki J, Puhl H, Jurgens G. The role of lipid peroxidation and antioxidants in oxidative modification of LDL. *Free Radic. Biol. Med.* 1992; 13:341-390). After a 4 h incubation period, the differential effects of these compounds could be clearly observed at a concentration of 3.0 µM. As compared to control lipid peroxidation (100% TBARS formation), TBARS formation for AML and ATM were 93.3 ± 6% and 65.6 ±7%, respectively. When combined, however, TBARS formation was only 29.3 ±6%, levels that were significantly (p<0.01) lower than that observed for either drug alone. The synergistic antioxidant effect of the combination persisted at the 6 h time point. These findings provide further evidence for synergistic activity consistent with that observed in membrane vesicles, as the drug combination produced a level of inhibition that substantially exceeded their expected additive effect. The antioxidant activity of vitamin E was similar to that observed for ATM alone in these experiments.

*Chemical Mechanisms for the Synergistic Activity of Amlodipine and Atorvastatin*

*Metabolite: The synergistic antioxidant activity observed for this drug combination in LDL and* reconstituted lipid membranes suggests that these compounds interact directly with each other to scavenge lipid radicals. Based on thermodynamic considerations (Figure 7), it is proposed that ATM reacts more quickly with lipid radicals (equation 1) than does AML, as described in equation 2. If these are the only two pathways available for these drugs when added together to the system, then their combined effect would only be additive. However, the combination of both compounds provides the possibility for a third pathway (equation 3), an alternative that is supported by the results of the peroxidation experiments in both LDL and lipid membranes. The combination of pathways 1 and 3 would produce a synergistic effect as it occurs more rapidly than pathway 2. Indeed, semi-empirical calculations suggest that reaction 3 is a favorable, exothermic process ( Δ H_{f} = -40.7 kJ/mol). Thus, the presence of the fast inhibitor (ATM) enables the slower inhibitor (AML) to remove free radicals more rapidly than if reacting with the lipid radicals on its own. The three pathways describing these interactions are as follows, in which LOO• represents a lipid radical:
(1) LOO• + ATM→ LOOH + ATM• *FAST*
(2) LOO• + AML → LOOH + AML• *SLOW*
(3) ATM• + AML → ATM+ AML • *FAST*

As a result of this synergy between AML and ATM, the recycled metabolite is now available for additional scavenging of lipid radicals. Overall, differences in the rates of inhibition between AML and ATM are based, in part, on the calculated enthalpies for these compounds and their respective radicals (Figure 7). The smaller (*i*.*e*., more negative) the ΔH_{f} value, the more favorable is hydrogen abstraction associated with radical formation. Once formed, the unpaired radical associated with radical species can be stabilized in resonance structures.

*Molecular Membrane Interactions of Amlodipine and Atorvastatin Metabolite*:

Small-angle x-ray diffraction approaches were used to examine the molecular membrane interactions of the AML and ATM combination. This highly quantitative technique provides direct information on the structure of the membrane lipid bilayer in the absence and presence of the drugs. It has been previously reported that AML has high affinity for membrane lipids (Kₚ>10³) under atherosclerotic conditions, as compared to other CCBs (Mason RP, Moisey DM, Shajenko L. Cholesterol alters the binding of Ca²⁺ channel blockers to the membrane lipid bilayer. *Mol. Pharmacol.* 1992; 41:315-321). The distinct lipophilicity of AML is attributed to its amphiphilic chemical structure that directs the molecule to an advantageous location in the membrane where it can then interfere with the propagation of free radicals by both biophysical and biochemical mechanisms, as previously described in detail by my laboratory (Mason RP, Leeds PR, Jacob RF, et al. Inhibition of excessive neuronal apoptosis by the calcium antagonist amlodipine and antioxidants in cerebellar granule cells. *J. Neurochem.* 1999; 72:1448-1456; Mason RP, Moisey DM, Shajenko L. Cholesterol alters the binding of Ca²⁺ channel blockers to the membrane lipid bilayer. *Mol. Pharmacol.* 1992; 41:315-321; Mason RP, Campbell SF, Wang SD, Herbette LG. Comparison of location and binding for the positively charged 1,4-dihydropyridine calcium channel antagonist amlodipine with uncharged drugs of this class in cardiac membranes. *Mol. Pharmacol.* 1989; 36:634-640; Mason RP, Walter MF, Trumbore MW, Olmstead Jr. EG, Mason PE. Membrane antioxidant effects of the charged dihydropyridine calcium antagonist amlodipine. *J. Mol. Cell. Cardiol*. 1999; 31:275-281) In membranes that are not enriched with cholesterol, amlodipine inhibited lipid peroxidation in a manner that could not be reproduced by other CCBs or the angiotensin converting enzyme (ACE)- inhibitor, captopril (Mason RP, Walter MF, Trumbore MW, Olmstead Jr. EG, Mason PE. Membrane antioxidant effects of the charged dihydropyridine calcium antagonist amlodipine. *J. Mol. Cell. Cardiol.* 1999; 31:275-281). In the same way, the chemical structure of the atorvastatin metabolite has amphiphilic properties that would enable the drug to interact strongly with the membrane lipid bilayer, as recently reported by my laboratory (Mason RP. Inhibition of oxidative damage to low density lipoproteins and isolated membranes by atorvastatin and its active metabolite. *J. Am. Coll. Cardiol.* 2000; 35:317A).

For these studies, the combination of AML and ATM were added to membrane vesicles reconstituted from cholesterol and phospholipid at a 0.5:1 mole ratio (Figure 8). X- ray diffraction analysis of the membrane samples produced strong and reproducible diffraction patterns for representative control and drug-containing samples. In the absence of drug, the overall membrane bilayer width, including surface hydration, was 55.5 Å with an intrabilayer headgroup separation of 44 Å. The addition of the two drugs together at a ratio of drug to phospholipid of 1:15 produced distinct changes in the structure and organization of the phospholipid bilayer, as compared to the drugs added separately (Figure 8). In the presence of the drug combination, the overall membrane bilayer width, including surface hydration, was reduced to 53.5 Å with an intrabilayer headgroup separation of 41 Å. Separately, the membrane bilayer widths of membranes containing AML and ATM alone were 54.8 Å and 58.0 Å, respectively (Figure 9). These structural findings provide direct evidence that the combination of these agents differentially modulate the structure of lipid molecules, as compared to their separate effects.

Direct subtraction of the membrane electron density profiles (Å versus electrons/ Å³) demonstrated large differences in lipid structure that could be attributed to the presence of the drugs (Figure 8). Specifically, the addition of the drug combination produced a broad increase in electron density associated with the upper hydrocarbon core/hydrated headgroup region of the membrane bilayer ± 11-21 Å from the center of the bilayer. This large increase in electron density distributed over 10 Å is attributed to the equilibrium location of the drugs in the membrane. Concomitant with this change was an observed disordering effect associated with the central hydrocarbon core region of the membrane, ± 0-11 Å. This decrease in electron density is due to an increase in molecular volume resulting from the insertion of the drug molecules into a region of high molecular density near the membrane hydrocarbon core/water interface. Thus, it can be concluded from these data that the insertion of the drug combination into the membrane bilayer alters the intermolecular packing constraints of the phospholipid molecules in a manner similar to that observed with either reducing cholesterol content or increasing sample temperature (Tulenko TN, Chen M, Mason PE, Mason RP. Physical effects of cholesterol on arterial smooth muscle membranes: Evidence of immiscible cholesterol domains and alterations in bilayer width during atherogenesis. *J. Lipid Res.* 1998; 39:947-956; Chang HM, Reitstetter R, Mason RP, Gruener R. Attenuation of channel kinetics and conductance by cholesterol: An interpretation using structural stress as a unifying concept. *J. Membr. Biol.* 1995; 143:51-63). Such changes in biophysical properties have been shown to interfere with the propagation of free radicals though the lipid bilayer matrix (McLean LR, Hagaman KA. Effect of lipid physical state on the rate of peroxidation of liposomes. *Free Radic. Biol. Med.* 1992; 12:113-119).

Separately, AML and ATM effected distinct changes in membrane structure, as compared to the drug combination, due to specific interactions with constituent phospholipid molecules (Figures 8 and 9). While the drug combination effected a 2 Å or 4% decrease (p<0.01) in overall membrane width, the ATM separately produced a 5% increase (p<0.01) in width (2.5 Å) while AML alone did not significantly alter membrane dimensions, including the intrabilayer headgroup separation. As compared to AML alone, ATM produced a larger reduction in hydrocarbon core electron density. This effect on membrane structure may contribute to its greater antioxidant potency, as compared to AML. The combination of AML and ATM effected a new site of interaction with the membrane lipid bilayer, in addition to their separate locations in the membrane (Figure 8).

Therefore, this invention is drawn to a pharmaceutical composition comprising amlodipine and atorvastatin metabolite. These individual pharmaceutical agents can be formulated in combination, or separately, in salts, forms, and dosages that produce maximal therapeutic responses. This combination therapy is designed to treat the various pathophysiological manifestations of arterial and related heart disease, including, but not limited to, hypertension, hyperlipidemia, atherosclerosis, arteriosclerosis, coronary artery disease, myocardial infarction, congestive heart failure, stroke, and angina pectoris. Specifically, this combination therapy will be designed to lower blood pressure and systemic lipid concentrations as well as the related pathophysiological results of the lack of their regulation, including, but not limited to, arterial weakening and plaque deposition. The effects of these individual agents on these various processes and events related to arterial and related heart disease, when used in combination can be additive and/or synergistic. *44*It will now be apparent to those skilled in the art that other embodiments, improvements, details, and uses can be made that are consistent with the letter and spirit of the foregoing disclosure and within the scope of this patent and the appended claims.

### Experimental Methods

Dilinoleoyl phosphatidylcholine (DLPC), 1 - palmitoyl-2-oleoyl phosphatidylcholine (POPC) and unesterified cholesterol were obtained from Avanti Polar Lipids Inc. (Alabaster, AL) and stored at -80°C. Aliquots of LDL (L-2139) from human plasma were obtained from Sigma Chemical Co. (St. Louis, MO). Sephadex G-25 M (PD- 10) columns were purchased from Pharmacia Biotech Inc. (Piscataway, NJ). Amlodipine besylate was obtained from Pfizer Central Research (Groton, CT) while the hydroxy metabolite of atorvastatin was provided by Parke Davis (Ann Arbor, MI). Vitamin E, Trolox (a vitamin E analog), lovastatin, and mevastatin were purchased from Sigma Chemical Co. (St. Louis, MO).

*Membrane Lipid Peroxidation Analysis:* The separate and combined dose-dependent antioxidant activities of these agents were examined in membranes enriched with PUFA prepared at a 0.5 cholesterol to phospholipid mole ratio (Mason RP, Walter MF, Trumbore MW, Olmstead Jr. EG, Mason PE. Membrane antioxidant effects of the charged dihydropyridine calcium antagonist amlodipine. *J. Mol. Cell. Cardiol.* 1999; 31:275-281). The lipid (dilinoleoyl phosphatidylcholine and cholesterol) used for these samples was dissolved in HPLC-grade chloroform (25.0 mg/ml). An aliquot of the lipids (1.0 mg) was added to individual glass 13 x 100-mm test tubes and chloroform was removed by shell-drying under a steady stream of N₂ gas. The lipids were dried down in the absence or presence of drug(s) dissolved in ethanol. Residual solvent was removed under vacuum while the samples were shielded from light. Membrane vesicles were produced by rapidly mixing the dried lipids at room temperature following addition of 1.0 ml HEPES buffered saline (0.5 mM HEPES and 154.0 mM NaCl, pH, 7.2). The final phospholipid concentration was 1.0 mg/ml buffer and the final concentration of drug ranged from 10.0 nM through 10.0 µM.

Membrane lipid peroxidation was carried out at 37°C in a shaking water bath without the addition of exogenous stimulants, as previously described in detail (Mason RP, Walter MF, Trumbore MW, Olmstead Jr. EG, Mason PE. Membrane antioxidant effects of the charged dihydropyridine calcium antagonist amlodipine. *J. Mol. Cell. Cardiol.* 1999; 31:275- 281). At various time intervals (24, 48, 65, 72h), an aliquot of lipid sample (10 to 100 µl) was removed before 25 µl of 5.0 mM ethylenediaminetetracetic acid (EDTA) and 20 µl of 35.0 mM butylated hydroxytoluene (BHT) was immediately added to the sample to stop the peroxidation reaction.

The extent of membrane lipid peroxidation was measured by the CHOD-Iodide assay as previously described in detail (El-Saadani M, Esterbauer H, el-Sayed M, Goher M, Nassar AY, Jurgens G. A spectrophotometric assay for lipid peroxides in serum lipoproteins using a commercially available reagent. *J. Lipid. Res.* 1989; 30:627-630). The quantity of I₃⁻ was measured from the following reaction in which *L* represents a phospholipid molecule:

LOOH + 2H⁺ + 2I⁻ ------> LOH + H₂O+ I₂

I₂ + I⁻ -------> I₃⁻

An aliquot of the membrane sample was removed at various time points and then added to 1.0 ml of CHOD color reagent (E.M. Science, Gibbstown, NJ) that includes 20.0 µM BHT, 24.0 µM EDTA, and 0.2% Triton-X. The sample was then covered with foil and allowed to incubate for 2 h in the absence of light before measuring the absorbance of the sample at 365 nm (ε=2.4 × 10⁴ M⁻¹cm⁻¹). The background sample was run along the test samples in triplicate and contains 76.7 µl of 0.652 mM HEPES, 20 µl of 5.0 mM EDTA and 3.3 µl of DDI water. The extent of lipid peroxidation was measured in triplicate for each drug concentration and compared to control samples that did not contain drug. The statistical significance of these experiments was assessed by the non-paired t-test. Significance was accepted at p<0.05.

*LDL Oxidation Determination:* In addition to lipid membranes, the antioxidant activity of AML and ATM was evaluated in human LDL. The EDTA content of the LDL samples obtained from human plasma was removed by gel filtration with PD-10 Sephadex G25-M filtration columns; PBS (nitrogen purged) was used as the eluent. The LDL samples (50 µg of protein/mL) were then preincubated with or without drug (3.0 µM) for 30 mm at 37°C. Oxidation of LDL was then induced by the addition of 10.0 µM CuSO₄. The time course of LDL oxidation, measured by TBARS formation, was followed for 6 h at 37°C (Mak IT, Kramer JH, Weglicki WB. Potentiation of free radical-induced lipid peroxidative injury to sarcolemmal membranes by lipid amphiphiles. *J. Biol. Chem.* 1986; 261:1153- 1157). LDL oxidation, as determined by the TBARS methods, followed sigmoidal curve kinetics with an initial lag phase followed by a sharp propagation and final plateau phase (Esterbauer H, Gebicki J, Puhl H, Jurgens G. The role of lipid peroxidation and antioxidants in oxidative modification of LDL. *Free Radic. Biol. Med.* 1992; 13:341-390). The protein content of the LDL was determined using the Coomassie Protein Plus assay kit from Pierce Chemical. A rapid and sensitive method for the quantitation of microgram quantities of protein utilizing the principle of protein-dye binding. *Anal. Biochem.* 1976; 72:248-254).

*Small angle X-ray diffraction analysis:* Small-angle X-ray diffraction analyses were used to directly examine the molecular membrane interactions of AML, and ATM. The lipids (1-palmitoyl-2-oleoyl phosphatidylcholine and cholesterol) used for these samples were dissolved in HPLC-grade chloroform (10.0 mg/ml). Membrane vesicles were produced from these lipids by the same method as described for the peroxidation experiments. The final phospholipid concentration was 5.0 mg/ml buffer and the mole ratio of drug to phospholipid was 1:15. Membrane samples were oriented for diffraction analysis by subjecting them to centrifugation as previously described (Chester DW, Herbette LG, Mason RP, Joslyn AF, Triggle DJ, Koppel DE. Diffusion of dihydropyridine calcium channel antagonists in cardiac sarcolemmal lipid multibilayers. *Biophys. J*. 1987; 52:1021-1030). Briefly, vesicles were placed in sedimentation cells that contained an aluminum foil substrate. The vesicles were sedimented in an SW-28 rotor (Beckman Instruments Fullerton, CA) at 35,000 x g for 1.5 h at 5°C. Following centrifugation, the supernatant was removed from the pellets and the samples were then mounted on to curved glass supports. Samples were placed in sealed canisters to control relative humidity and temperature during the diffraction experiments, as previously described in detail (Chester DW, Herbette LG, Mason RP, Joslyn AF, Triggle DJ, Koppel DE. Diffusion of dihydropyridine calcium channel antagonists in cardiac sarcolemmal lipid multibilayers. *Biophys. J.* 1987; 52: 1021-1030).

X-ray diffraction experiments were conducted by aligning the samples at grazing incidence with respect to a collimated, nickel-filtered monochromatic X-ray source (CuK_{α}1.54 Å) produced by a high-brilliance rotating anode microfocus generator (Rigaku Rotaflex RU-200, Danvers, MA). The fixed geometry beam line consisted of a single, nickel-coated Franks mirror to define a line source where K_{α₁} and K_{α₂} are unresolved. The diffraction data were collected on a one-dimensional, positi on-sensitive electronic detector (Innovative Technologies, Newburyport, MA) placed at a distance of 150 mm from the sample. Each meridional diffraction peak was Lorentz- and background-corrected, as previously described (Mason RP, Gonye GE, Chester DW, Herbette LG. Partitioning and location of Bay K 8644, 1,4- dihydropyridine calcium channel agonist, in model and biological membranes. *Biophys. J.* 1989; 55:769-778). The phases for the four-order data were determined by swelling analysis (Moody MF. X-ray diffraction pattern of nerve myelin: A method for determining the phases. Science 1963; 142:1173-117). Fourier transformations of the data were generated from the diffraction data with Origin software (Microcal Software, Northampton, MA).

## Claims

1. A pharmaceutical composition comprising:
(a) an effective amount of amlodipine;
(b) an effective amount of hydroxylated atorvastatin metabolite (ATM); and
(c) pharmaceutically acceptable formulation agents.

2. The pharmaceutical composition of claim 1 wherein the amlodipine is provided as a therapeutically effective derivative of amlodipine.

3. The pharmaceutical composition of claim 2 wherein the therapeutically effective derivative of amlodipine comprises amlodipine besylate.

4. The pharmaceutical composition of any of claims 1 to 3 for use in reducing the risk of arterial and related heart disease.

5. The pharmaceutical composition of claim 4 wherein said arterial and related heart disease is selected from the group consisting of hypertension, hyperlipidemia, atherosclerosis, arteriosclerosis, coronary artery disease, myocardial infarction, congestive heart failure, stroke, and angina pectoris.

6. The pharmaceutical composition of any of claims 1 to 3 for use in lowering blood pressure.

7. The pharmaceutical composition of claim 6 wherein said pharmaceutical composition lowers blood pressure to a level consistent with a reduced risk of arterial and related heart disease.

8. The pharmaceutical composition of claim 6 wherein said pharmaceutical composition lowers blood pressure to a level statistically equivalent to normal.

9. The pharmaceutical composition of any of claims 1 to 3 for use in lowering systemic lipid concentrations.

10. The pharmaceutical composition of claim 9 wherein said pharmaceutical composition lowers systemic lipid concentrations to a level consistent with a reduced risk of arterial and related heart disease.

11. The pharmaceutical composition of claim 9 wherein said pharmaceutical composition lowers systemic lipid concentrations to a level statistically equivalent to normal.

12. The pharmaceutical composition of any of claims 1 to 3 for use in lowering blood pressure and systemic lipid concentrations.

13. The pharmaceutical composition of claim 12 wherein said pharmaceutical composition lowers blood pressure and systemic lipid concentrations to a level consistent with a reduced risk of arterial and related heart disease.

14. The pharmaceutical composition of claim 12 wherein said pharmaceutical composition lowers blood pressure and systemic lipid concentrations to a level statistically equivalent to normal.

15. The pharmaceutical composition of any of claims 1 to 3 for use in concomitantly lowering blood pressure and systemic lipid concentrations.

16. The pharmaceutical composition of claim 15 wherein said pharmaceutical composition concomitantly lowers blood pressure and systemic lipid concentrations to a level consistent with a reduced risk of arterial and related heart disease.

17. The pharmaceutical composition of claim 15 wherein said pharmaceutical composition concomitantly lowers blood pressure and systemic lipid concentrations to a level statistically equivalent to normal.

18. The pharmaceutical composition of any of claims 1 to 3 for use in synergistically lowering blood pressure and systemic lipid concentrations.

19. The pharmaceutical composition of claim 18 wherein said pharmaceutical composition synergistically lowers blood pressure and systemic lipid concentrations to a level consistent with a reduced risk of arterial and related heart disease.

20. The pharmaceutical composition of claim 18 wherein said pharmaceutical composition synergistically lowers blood pressure and systemic lipid concentrations to a level statistically equivalent to normal.

21. The pharmaceutical composition of any of claims 15-17 and 18-20 wherein the concomitant lowering of blood pressure and systemic lipid concentrations results at least partially from reduced lipid oxidation.

22. The pharmaceutical composition of any of claims 1-21 wherein amlodipine and the atorvastatin metabolite are administered in the same therapeutic or as separate therapeutics or at the same time or at different times.

23. Use of a combination of amlodipine and hydroxylated atorvastatin metabolite for the preparation of a medicament for use in:
(a) treating arterial and related heart disease; or
(b) substantially inhibiting lipid peroxidation in LDL and lipid membranes; or
(c) substantially inhibiting lipid oxidation and lowering blood pressure and systemic lipid concentrations; or
(d) synergistically inhibiting lipid oxidation.

24. The use of claim 23 wherein amlodipine comprises a therapeutically effective derivative of amlodipine.

25. The use of claim 24 wherein the therapeutically effective derivative of amlodipine comprises amlodipine besylate.

26. The use of any of claims 23-25 wherein said medicament lowers blood pressure and systemic lipid concentrations to a level consistent with a reduced risk of arterial and related heart disease.

27. The use of any of claims 23-25 wherein said medicament lowers blood pressure and systemic lipid concentrations to a level statistically equivalent to normal.

28. The use of any of claims 23-27 wherein the lowering of blood pressure and systemic lipid concentrations results at least partially from reduced lipid oxidation.

29. The use of any of claims 23-25 wherein said medicament inhibits lipid oxidation to an extent consistent with a reduced risk of arterial and related heart disease.

30. The use of any of claims 23-26 or 29 wherein said arterial and related heart disease is selected from the group consisting of hypertension, hyperlipidemia, atherosclerosis, arteriosclerosis, coronary artery disease, myocardial infarction, congestive heart failure, stroke, and angina pectoris.

31. The use of any of claims 23-25 wherein said medicament synergistically lowers blood pressure and systemic lipid concentrations to a level statistically equivalent to normal.

32. The use of claim 31 wherein the synergistic lowering of blood pressure and systemic lipid concentrations results at least partially from reduced lipid oxidation.

33. The use of any of claims 23-32 wherein amlodipine and the atorvastatin metabolite are administered in the same therapeutic or as separate therapeutics or at the same time or at different times.

## Patentansprüche

1. Pharmazeutische Zusammensetzung, umfassend:
(a) eine wirksame Amlodipin-Menge;
(b) eine wirksame Menge eines hydroxylierten Atorvastatin-Metabolits (ATM); und
(c) pharmazeutisch verträgliche Formulierungsmittel.

2. Pharmazeutische Zusammensetzung nach Anspruch 1, worin das Amlodipin als ein therapeutisch wirksames Amlodipin-Derivat bereitgestellt wird.

3. Pharmazeutische Zusammensetzung nach Anspruch 2, worin das therapeutisch wirksame Amlodipin-Derivat Amlodipin-Besylat umfasst.

4. Pharmazeutische Zusammensetzung nach einem der Ansprüche 1 bis 3 zur Anwendung bei der Reduktion des Risikos für eine arterielle und verwandte Herzkrankheit.

5. Pharmazeutische Zusammensetzung nach Anspruch 4, worin die genannte arterielle und verwandte Herzkrankheit aus der Gruppe ausgewählt ist, bestehend aus Hypertonie, Hyperlipidämie, Atherosklerose, Arteriosklerose, koronarer Herzkrankheit, Myokardinfarkt, kardialer Stauungsinsuffizienz, Schlaganfall und Angina pectoris.

6. Pharmazeutische Zusammensetzung nach einem der Ansprüche 1 bis 3 zur Anwendung bei der Senkung des Blutdrucks.

7. Pharmazeutische Zusammensetzung nach Anspruch 6, worin die genannte pharmazeutische Zusammensetzung den Blutdruck auf eine Höhe senkt, die mit einem reduzierten Risiko für eine arterielle und verwandte Herzkrankheit konsistent ist.

8. Pharmazeutische Zusammensetzung nach Anspruch 6, worin die genannte pharmazeutische Zusammensetzung den Blutdruck auf eine Höhe senkt, die statistisch dem Normalbereich entspricht.

9. Pharmazeutische Zusammensetzung nach einem der Ansprüche 1 bis 3 zur Anwendung bei der Senkung der systemischen Lipikonzentrationen.

10. Pharmazeutische Zusammensetzung nach Anspruch 9, worin die genannte pharmazeutische Zusammensetzung die systemischen Lipidkonzentrationen auf eine Höhe senkt, die mit einem reduzierten Risiko für eine arterielle und verwandte Herzkrankheit konsistent ist.

11. Pharmazeutische Zusammensetzung nach Anspruch 9, worin die genannte pharmazeutische Zusammensetzung die systemischen Lipikonzentrationen auf eine Höhe senkt, die statistisch dem Normalbereich entspricht.

12. Pharmazeutische Zusammensetzung nach einem der Ansprüche 1 bis 3 zur Anwendung bei der Senkung des Blutdrucks und der systemischen Lipidkonzentrationen.

13. Pharmazeutische Zusammensetzung nach Anspruch 12, worin die genannte pharmazeutische Zusammensetzung den Blutdruck und die systemischen Lipidkonzentrationen auf eine Höhe senkt, die mit einem reduzierten Risiko für eine artererielle und verwandte Herzkrankheit konsistent ist.

14. Pharmazeutische Zusammensetzung nach Anspruch 12, worin die genannte pharmazeutische Zusammensetzung den Blutdruck und die systemischen Lipidkonzentrationen auf eine Höhe senkt, die statistisch dem Normalbereich entspricht.

15. Pharmazeutische Zusammensetzung nach einem der Ansprüche 1 bis 3 zur Anwendung bei der gleichzeitigen Senkung des Blutdrucks und der systemischen Lipidkonzentrationen.

16. Pharmazeutische Zusammensetzung nach Anspruch 15, worin die genannte pharmazeutische Zusammensetzung gleichzeitig den Blutdruck und die systemischen Lipidkonzentrationen auf eine Höhe senkt, die mit einem reduzierten Risiko für eine arterielle und verwandte Herzkrankheit konsistent ist.

17. Pharmazeutische Zusammensetzung nach Anspruch 15, worin die genannte pharmazeutische Zusammensetzung gleichzeitig den Blutdruck und die systemischen Lipidkonzentrationen auf eine Höhe senkt, die statistisch dem Normalbereich entspricht.

18. Pharmazeutische Zusammensetzung nach einem der Ansprüche 1 bis 3 zur Anwendung bei der synergistischen Senkung des Blutdrucks und der systemischen Lipidkonzentrationen.

19. Pharmazeutische Zusammensetzung nach Anspruch 18, worin die genannte pharmazeutische Zusammensetzung den Blutdruck und die systemischen Lipidkonzentrationen synergistisch auf eine Höhe senkt, die mit einem reduzierten Risiko für eine arterielle und verwandte Herzkrankheit konsistent ist.

20. Pharmazeutische Zusammensetzung nach Anspruch 18, worin die genannte pharmazeutische Zusammensetzung den Blutdruck und die systemischen Lipidkonzentrationen synergistisch auf eine Höhe senkt, die statistisch dem Normalbereich entspricht.

21. Pharmazeutische Zusammensetzung nach einem der Ansprüche 15 bis 17 und 18 bis 20, worin die gleichzeitige Senkung des Blutdrucks und der systemischen Lipidkonzentrationen mindestens teilweise aus der reduzierten Lipidoxidation resultiert.

22. Pharmazeutische Zusammensetzung nach einem der Ansprüche 1 bis 21, worin Amlodipin und der Atorvastatin-Metabolit im gleichen Therapeutikum oder als getrennte Therapeutika oder gleichzeitig oder zu verschiedenen Zeiten verabreicht werden.

23. Verwendung einer Kombination aus Amlodipin und hydroxyliertem Atorvastatin-Metabolit zur Herstellung eines Arzneimittels zur Anwendung bei:
(a) der Behandlung der arteriellen oder verwandten Herzkrankheit; oder
(b) der weitgehenden Hemmung der Lipidperoxidation in LDL- und Lipidmembranen; oder
(c) der weitgehenden Hemmung der Lipidoxidation und Senkung des Blutdrucks und der systemischen Lipidkonzentrationen; oder
(d) der synergistischen Hemmung der Lipidoxidation.

24. Verwendung nach Anspruch 23, worin Amlodipin ein therapeutisch wirksames Amlodipin-Derivat umfasst.

25. Verwendung nach Anspruch 24, worin das therapeutisch wirksame Amlodipin-Derivat Amlodipin-Besylat umfasst.

26. Verwendung nach einem der Ansprüche 23 bis 25, worin das genannte Arzneimittel den Blutdruck und die systemischen Lipidkonzentrationen auf eine Höhe senkt, die mit einem reduzierten Risiko für eine arterielle und verwandte Herzkrankheit konsistent ist.

27. Verwendung nach einem der Ansprüche 23 bis 25, worin das genannte Arzneimittel den Blutdruck und die systemischen Lipidkonzentrationen auf eine Höhe senkt, die statistisch dem Normalbereich entspricht.

28. Verwendung nach einem der Ansprüche 23 bis 27, worin das Senken des Blutdrucks und der systemischen Lipidkonzentrationen mindestens teilweise aus der reduzierten Lipidoxidation resultiert.

29. Verwendung nach einem der Ansprüche 23 bis 25, worin das genannte Arzneimittel die Lipidoxidation bis zu einem Grad hemmt, der mit einem reduzierten Risiko für eine arterielle und verwandte Herzkrankheit konsistent ist.

30. Verwendung nach einem der Ansprüche 23 bis 26 oder 29, worin die genannte arterielle und verwandte Herzkrankheit aus der Gruppe ausgewählt ist, bestehend aus Hypertonie, Hyperlipidämie, Atherosklerose, Arteriosklerose, der koronaren Herzkrankheit, dem Myokardinfarkt, der kardialen Stauungsinsuffizienz, Schlaganfall und Angina pectoris.

31. Verwendung nach einem der Ansprüche 23 bis 25, worin das genannte Arzneimittel den Blutdruck und die systemischen Lipidkonzentrationen synergistisch auf eine Höhe senkt, die statistisch dem Normalbereich entspricht.

32. Verwendung nach Anspruch 31, worin die synergistische Senkung des Blutdrucks und der systemischen Lipidkonzentrationen mindestens teilweise aus der reduzierten Lipidoxidation resultiert.

33. Verwendung nach einem der Ansprüche 23 bis 32, worin Amlodipin und der Atorvastatin-Metabolit im gleichen Therapeutikum oder als getrennte Therapeutika oder gleichzeitig oder zu verschiedenen Zeiten verabreicht werden.

## Revendications

1. Composition pharmaceutique comprenant :
(a) une quantité efficace d'amlodipine ;
(b) une quantité efficace d'un métabolite hydroxylé de l'atorvastatine (MHA) ; et
(c) des agents de formulation pharmaceutiquement acceptables.

2. Composition pharmaceutique selon la revendication 1 dans laquelle l'amlodipine est fournie en tant que dérivé thérapeutiquement efficace de l'amlodipine.

3. Composition pharmaceutique selon la revendication 2 dans laquelle le dérivé thérapeutiquement efficace de l'amlodipine comprend le bésylate d'amlodipine.

4. Composition pharmaceutique selon n'importe laquelle des revendications 1 à 3 utilisée pour réduire le risque de maladie artérielle et cardiaque connexe.

5. Composition pharmaceutique selon la revendication 4 dans laquelle ladite maladie artérielle et cardiaque connexe est sélectionnée parmi le groupe consistant en l'hypertension, l'hyperlipidémie, l'athérosclérose, l'artériosclérose, les maladies artérielles coronaires, l'infarctus du myocarde, l'insuffisance cardiaque congestive, les AVC, et l'angine de poitrine.

6. Composition pharmaceutique selon n'importe laquelle des revendications 1 à 3 utilisée pour abaisser la pression sanguine.

7. Composition pharmaceutique selon la revendication 6 dans laquelle ladite composition pharmaceutique abaisse la pression sanguine à un niveau compatible avec un risque réduit de maladie artérielle et cardiaque connexe.

8. Composition pharmaceutique selon la revendication 6 dans laquelle ladite composition pharmaceutique abaisse la pression sanguine à un niveau statistiquement équivalent à la normale.

9. Composition pharmaceutique selon n'importe laquelle des revendications 1 à 3 utilisée pour abaisser les concentrations systémiques en lipides.

10. Composition pharmaceutique selon la revendication 9, ladite composition pharmaceutique abaissant les concentrations systémiques en lipides à un niveau compatible avec un risque réduit de maladie artérielle et cardiaque connexe.

11. Composition pharmaceutique selon la revendication 9, ladite composition pharmaceutique abaissant les concentrations systémiques en lipides à un niveau statistiquement équivalent à la normale.

12. Composition pharmaceutique selon n'importe laquelle des revendications 1 à 3 destinée à abaisser la pression sanguine et les concentrations systémiques en lipides.

13. Composition pharmaceutique selon la revendication 12, ladite composition pharmaceutique abaissant la pression sanguine et les concentrations systémiques en lipides à un niveau compatible avec un risque réduit de maladie artérielle et cardiaque connexe.

14. Composition pharmaceutique selon la revendication 12, ladite composition pharmaceutique abaissant la pression sanguine et les concentrations systémiques en lipides à un niveau statistiquement équivalent à la normale.

15. Composition pharmaceutique selon n'importe laquelle des revendications 1 à 3 utilisée pour abaisser de manière concomitante la pression sanguine et les concentrations systémiques en lipides.

16. Composition pharmaceutique selon la revendication 15, ladite composition pharmaceutique abaissant de manière concomitante la pression sanguine et les concentrations systémiques en lipides à un niveau compatible avec un risque réduit de maladie artérielle et cardiaque connexe.

17. Composition pharmaceutique selon la revendication 15, ladite composition pharmaceutique abaissant de manière concomitante la pression sanguine et les concentrations systémiques en lipides à un niveau statistiquement équivalent à la normale.

18. Composition pharmaceutique selon n'importe laquelle des revendications 1 à 3 utilisée pour abaisser de manière synergique la pression sanguine et les concentrations systémiques en lipides.

19. Composition pharmaceutique selon la revendication 18, ladite composition pharmaceutique abaissant de manière synergique la pression sanguine et les concentrations systémiques en lipides à un niveau compatible avec un risque réduit de maladie artérielle et cardiaque connexe.

20. Composition pharmaceutique selon la revendication 18, ladite composition pharmaceutique abaissant de manière synergique la pression sanguine et les concentrations systémiques en lipides à un niveau statistiquement équivalent à la normale.

21. Composition pharmaceutique selon n'importe laquelle des revendications 15 à 17 et 18 à 20 dans laquelle l'abaissement concomitant de pression sanguine et des concentrations systémiques en lipides résulte au moins partiellement d'une oxydation réduite des lipides.

22. Composition pharmaceutique selon n'importe laquelle des revendications 1 à 21 dans laquelle l'amlodipine et le métabolite de l'atorvastatine sont administrés dans le même traitement ou en tant que traitements séparés ou en même temps ou à des temps différents.

23. Utilisation d'une combinaison d'amlodipine et d'un métabolite hydroxylé de l'atorvastatine pour la préparation d'un médicament utilisé pour :
(a) traiter les maladies artérielles et cardiaques connexes ; ou
(b) inhiber nettement la peroxydation des lipides dans les LDL et les membranes lipidiques ; ou
(c) inhiber nettement l'oxydation des lipides et abaisser la pression sanguine et les concentrations systémiques en lipides ; ou
(d) inhiber de manière synergique l'oxydation des lipides.

24. Utilisation selon la revendication 23, l'amlodipine comprenant un dérivé thérapeutiquement efficace de l'amlodipine.

25. Utilisation selon la revendication 24, le dérivé thérapeutiquement efficace de l'amlodipine comprenant le bésylate d'amlodipine.

26. Utilisation selon n'importe laquelle des revendications 23 à 25, ledit médicament abaissant la pression sanguine et les concentrations systémiques en lipides à un niveau compatible avec un risque réduit de maladie artérielle et cardiaque connexe.

27. Utilisation selon n'importe laquelle des revendications 23 à 25, ledit médicament abaissant la pression sanguine et les concentrations systémiques en lipides à un niveau statistiquement équivalent à la normale.

28. Utilisation selon n'importe laquelle des revendications 23 à 27, l'abaissement de la pression sanguine et des concentrations systémiques en lipides résultant au moins en partie d'une oxydation réduite des lipides.

29. Utilisation selon n'importe laquelle des revendications 23 à 25, ledit médicament inhibant l'oxydation des lipides à un niveau compatible avec un risque réduit de maladie artérielle et cardiaque connexe.

30. Utilisation selon n'importe laquelle des revendications 23 à 26 ou 29, ladite maladie artérielle et cardiaque connexe étant sélectionnée parmi le groupe consistant en l'hypertension, l'hyperlipidémie, l'athérosclérose, l'artériosclérose, les maladies artérielles coronaires, l'infarctus du myocarde, l'insuffisance cardiaque congestive, les AVC, et l'angine de poitrine.

31. Utilisation selon n'importe laquelle des revendications 23 à 25, ledit médicament abaissant de manière synergique la pression sanguine et les concentrations systémiques en lipides à un niveau statistiquement équivalent à la normale.

32. Utilisation selon la revendication 31, l'abaissement synergique de la pression sanguine et des concentrations systémiques en lipides résultant au moins en partie d'une oxydation réduite des lipides.

33. Utilisation selon n'importe laquelle des revendications 23 à 32, l'amlodipine et le métabolite de l'atorvastatine étant administrés dans le même traitement ou en tant que traitements séparés ou en même temps ou à des temps différents.
